# FASCICULE DE BREVET EUROPEEN

(11) **EP 3 528 755 B1**
(45) Date de publication et mention de la délivrance du brevet: **05.01.2022**
(21) Numéro de dépôt: 17797678.4
(22) Date de dépôt: 18.10.2017
(51) Int. Cl.: A61F 5/055, A41D 13/05, A61F 5/01

(54) **DISPOSITIF APPUITETE A MOYENS ELASTIQUES DE RAPPEL VERS UNE POSITION NOMINALE**
KOPFSTÜTZENDE VORRICHTUNG MIT ELASTISCHEN MITTELN ZUR RÜCKKEHR IN EINE SOLLPOSITION
HEAD-SUPPORT DEVICE WITH ELASTIC MEANS TO RETURN TO A NOMINAL POSITION

(30) Priorité: 18.10.2016 FR 1601511
(43) Date de publication de la demande: 28.08.2019
(73) Titulaire: Briant, Paul, 15270 Trémouille (FR)
(72) Inventeur: Briant, Paul, 15270 Trémouille (FR)
(74) Mandataire: Fédit-Loriot
(86) Numéro de dépôt international: PCT/FR2017/052870
(87) Numéro de publication internationale: WO 2018/073540

(56) Documents cités:
- FR-A1- 3 019 973
- US-B1- 6 308 345

## Description

L'invention a pour objet un dispositif appui-tête du type conçu principalement pour améliorer le confort d'utilisateurs ayant à se tenir la tête en extension, c'est-à-dire inclinée en arrière, pendant de longues périodes et/ou de manière fréquemment réitérée.

Dans des situations où un individu doit maintenir la tête inclinée vers l'arrière pendant longtemps, pendant plusieurs heures parfois, ou quand il est amené à fréquemment regarder vers le haut, il s'ensuit des douleurs, voire des lésions, au niveau du cou et des vertèbres. De fait les mouvements de la tête en extension vers l'arrière sont particulièrement inconfortables et lourds de conséquences à cause de la compression créée sur l'arrière des vertèbres cervicales.

De telles situations peuvent se rencontrer par exemple dans le domaine de l'escalade pour l'assurance d'un grimpeur réalisée par une personne qui doit garder un contact visuel permanent depuis le sol sur le grimpeur pour assurer au maximum sa sécurité. Ce genre de situations se rencontrent surtout chez les travailleurs dans de nombreux domaines, par exemple pour les employés du bâtiment, quand ils ont notamment à appliquer des enduits ou des peintures en hauteur, en plafond en particulier, ou pour des techniciens qui interviennent sur des lignes aériennes de circuits électriques ou téléphoniques, ou encore pour les mécaniciens qui interviennent sous des véhicules. L'expérience montre que le travail en position couché sur le dos est particulièrement pénible et qu'il est particulièrement délicat d'assurer un confort satisfaisant pour les personnes travaillant dans ces conditions.

Il a déjà été proposé des dispositifs appui-tête comportant des moyens de liaison élastique entre un support dorsal fixe se disposant sur le dos du porteur et un repose-tête d'appui sur la base de la tête du porteur, donc au niveau de l'os occipital. On citera plus particulièrement le document de brevet FR 3 019 973, notamment par ce qu'il décrit d'un montage à inclinaison élastique du repose-tête sur le support dorsal. Le document US 6 308 345 décrit un dispositif similaire. Le premier de ces documents propose en plus un mode de montage axial du repose-tête sur le support dorsal qui est réglable, mais rigide.

Dans le but d'apporter encore plus de confort à l'utilisateur et de s'adapter à des conditions plus sévères, en évitant fatigue et troubles musculo-squelettiques, dans le souci aussi d'améliorer les conditions de fabrication du dispositif dans son ensemble et à apporter plus de souplesse à son adaptation à chaque porteur particulier, la présente invention propose pour l'essentiel d'équiper le dispositif appui-tête de moyens d'élongation élastique du support dorsal sollicitant axialement le repose-tête vers une position nominale de repos à l'encontre des mouvements de la tête du porteur tendant à l'enfoncer dans le support dorsal.

Grâce à des moyens de rappel élastique du repose-tête vers sa position nominale par rapport au support dorsal qui agissent simultanément en antagonisme avec les mouvements de basculement du repose-tête dans son montage articulé sur le support dorsal et en antagonisme avec les mouvements de translation longitudinale du repose-tête tendant à raccourcir le support dorsal, l'invention permet un suivi permanent des mouvements de la tête du porteur en extension par appui sans glissement au niveau de l'occiput (l'os occipital du crâne). La qualité du suivi sans glissement qui peut ainsi être obtenue quand le repose-tête est monté à la fois élastiquement coulissant en translation longitudinale le long du support dorsal et élastiquement articulé sur le support dorsal en basculement transversal, confère au dispositif suivant l'invention un confort que l'on ne savait obtenir par les dispositifs dont les moyens de rappel élastique n'avaient d'action que sur le mouvement de basculement du repose-tête en correspondance avec les variations d'inclinaison de la tête vers l'arrière dans ses mouvements d'extension. Or dans la pratique, on peut observer que la tête en extension tend à s'enfoncer de haut en bas en même temps qu'elle s'incline d'avant en arrière.

Dans les formes de mise en oeuvre préférées de l'invention, il est en outre prévu des moyens de réglage desdits moyens de rappel élastique permettant de définir la position nominale du repose-tête par rapport au support dorsal en correspondance avec une position de repos initiale jugée naturelle pour la tête de l'utilisateur. De tels moyens de réglage sont avantageusement accessibles à l'utilisateur, y compris quand il porte le dispositif appui-tête et le cas échéant au cours de son travail sur un chantier. Ils sont avantageusement conçus pour agir tant en réglage de l'inclinaison initiale du repose-tête sur le support dorsal (en correspondance avec un port de tête en extension naturelle) qu'en réglage de la position longitudinale du montage articulé sur le support dorsal (en correspondance avec une hauteur de cou jugée le plus confortable par l'utilisateur), donc en réglage d'une élongation initiale du support dorsal.

C'est ainsi notamment que les moyens de rappel élastique du repose-tête par rapport au support dorsal peuvent au mieux comporter, d'une part un ressort de torsion prenant appui sur le repose-tête (notamment une pièce de celui-ci formant raidisseur quand elle est enveloppée de matière souple pour des questions de confort de contact) et sur une portée supérieure d'un coulisseau monté à coulissement dans le corps principal du support dorsal, et d'autre part un ressort de compression maintenu dans un alésage guide interne au coulisseau et tendant à déployer celui-ci par allongement du support dorsal.

Avantageusement, le dispositif selon l'invention, présente des moyens de réglage de ses caractéristiques dimensionnelles qui permettent de l'ajuster à la morphologie de l'utilisateur et/ou au confort souhaité, spécialement en ce qui concerne sa hauteur de cou et sa souplesse cervicale. En particulier, on peut notamment prévoir à cet effet des moyens de réglage de la position initiale du coulisseau en dépassement longitudinal par rapport au support dorsal lorsque la compression du ressort entre eux est supposée nulle, ou du moins négligeable pour l'utilisateur tenant sa tête naturellement, en position de repos.

Le dispositif appui-tête suivant l'invention peut avantageusement être équipé de divers moyens de réglage permettant un réglage plus fin des conditions présidant à son utilisation, avec pour objectif de l'adapter au cas par cas d'une part à l'utilisateur porteur du dispositif, d'autre part à chaque situation d'emploi, en améliorant à chaque instant le ressenti de l'utilisateur. C'est ainsi que l'on peut prévoir notamment des moyens de réglage des effets de rappel assurés par chacun des deux ressorts, lesquels moyens seront de préférence accessibles au porteur en cours d'emploi du dispositif, sans qu'il ait besoin de retirer celui-ci ou de changer de position. De ce point de vue, il est particulièrement avantageux de prévoir des moyens de réglage de la raideur du ressort de compression qui constitue les moyens d'élongation élastique du support dorsal.

De préférence selon l'invention, le dispositif appui-tête comprend, en plus de la partie mécanique, une partie harnais constituée par un harnais fait de sangles attachées les unes aux autres en divers points d'attache qui sont de préférence réglables, les uns au départ en fonction de la morphologie propre de la personne qui utilisera le dispositif, les autres par l'utilisateur lui-même en fonction du confort qu'il recherche dans chaque position et chaque application particulière. Les réglages à effectuer par l'utilisateur alors qu'il porte déjà le dispositif sur lui peuvent avantageusement se faire en agissant sur la longueur des sangles.

Le rôle de ce harnais est de maintenir le support dorsal de la partie mécanique fermement en place sur le haut du torse dans le dos de l'utilisateur porteur et de reporter de manière équilibrée les efforts à supporter depuis cette partie mécanique, notamment en les ramenant partiellement sur la poitrine du porteur de manière à soulager son dos. Un harnais à sangles particulièrement efficace en soulagement des lombaires, et surtout en soulagement des cervicales dans les situations où la tête du porteur se place en extension, avec en conséquence un effet de prévention de l'arthrose, comporte des sangles s'étendant des épaules à des sangles de tour de cuisse qui se croisent sur la poitrine.

L'invention sera maintenant plus complètement décrite dans le cadre de caractéristiques préférées et de leurs avantages, en faisant référence à des modes de réalisation particuliers du dispositif appui-tête suivant l'invention, illustré par les figures 1 à 6 dans lesquelles :
- La figure 1 est une vue en perspective qui représente un dispositif appui-tête suivant l'invention décrit en ses éléments mécaniques, sans le harnais à sangles ;
- La figure 2 représente un détail de la figure 1, dans une vue sous un autre angle, de l'extrémité haute de la partie mécanique ;
- La figure 3 représente une variante de réalisation de la partie mécanique du dispositif dans une vue en coupe longitudinale ;
- La figure 4 montre le même dispositif en vue de profil ;
- La figure 5 montre comment est constituée la partie harnais du dispositif appui-tête selon l'invention, qui est ici représentée quand le harnais est attaché sur un utilisateur porteur, dans une vue vers la face antérieure du torse de l'utilisateur ;
- La figure 6 illustre schématiquement l'utilisateur portant le dispositif appui-tête dans une vue de profil.

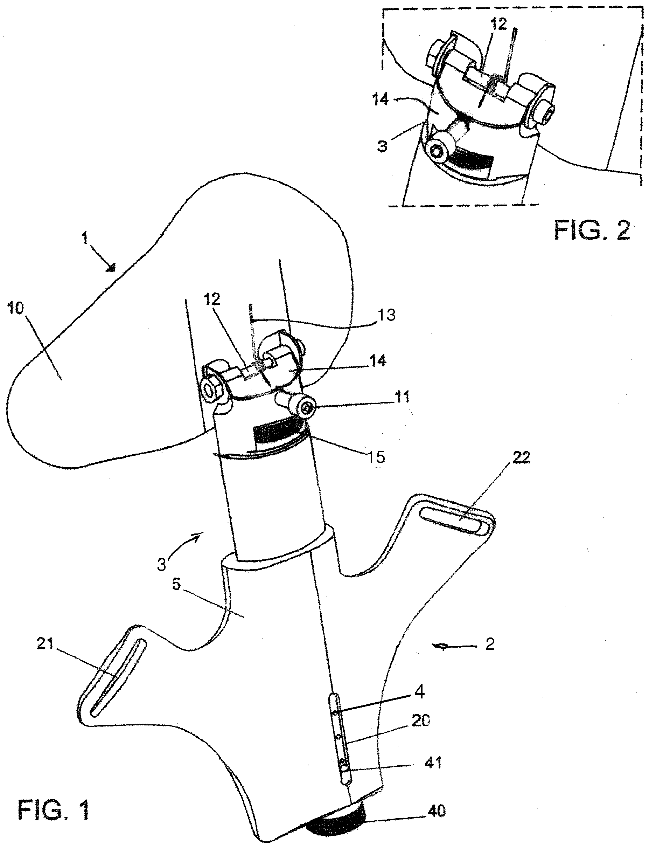

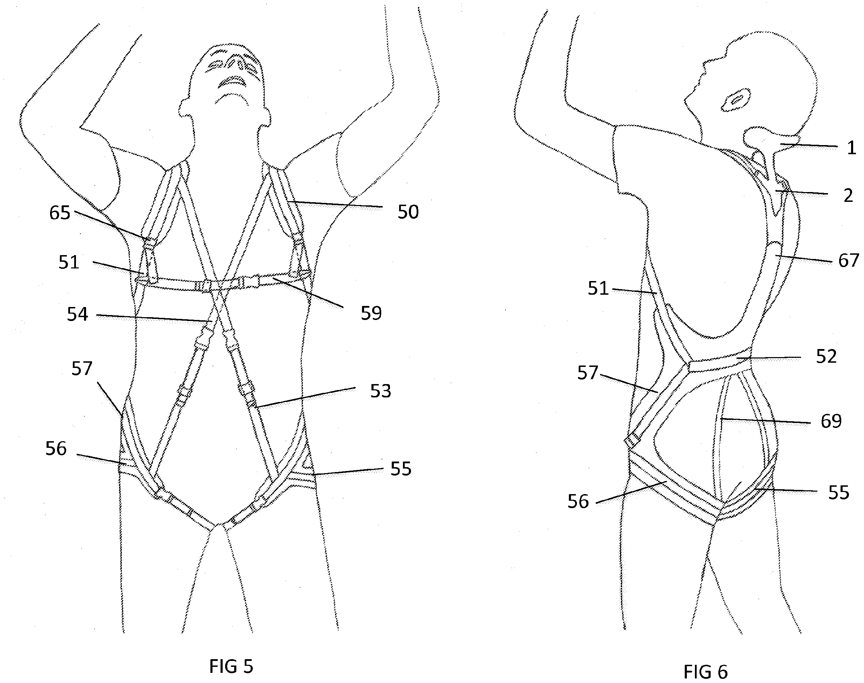

Conformément à l'invention, le dispositif appui-tête décrit ici, tel qu'il est illustré sur les figures, comporte essentiellement une partie mécanique et une partie harnais, réalisée à partir de sangles s'attachant les unes aux autres. Les éléments de la partie mécanique (fig. 1 et 3-4) comprennent principalement un repose-tête 1 qui est monté articulé en basculement à l'extrémité d'un coulisseau 3 lui-même monté coulissant longitudinalement par rapport à un élément support de l'ensemble mécanique qui constitue ce que l'on appelle ici un support dorsal 2, au motif qu'il se porte fixe en haut du dos de l'utilisateur porteur du dispositif appui-tête, comme on le voit sur les figures 5 et 6. Conformément à l'invention, tant le montage à articulation que le montage à coulissement sont associés à des moyens de rappel élastique qui sollicitent le repose-tête vers une position nominale correspondant à un port de tête naturel de l'utilisateur au repos.

Dans la présente description, les directions et orientations sont définies par référence au porteur debout, la verticale étant le long de sa colonne vertébrale, et avant et arrière correspondant à la face ventrale et à la face dorsale du porteur respectivement.

Le repose-tête 1 est configuré pour épouser la forme bombée sur lequel l'arrière de la tête d'un utilisateur vient en appui notamment lorsque la tête est inclinée vers l'arrière. Plus spécifiquement le repose-tête est essentiellement constitué par une pièce principale, ou plaque interne 10, réceptrice de la tête du porteur ; c'est sur elle que vient appuyer la base occipitale du crâne. Cette pièce est rigide, mais avantageusement recouverte d'une enveloppe en matière souple, telle une mousse de polymère organique, pour des questions de confort de contact. Elle est de préférence réalisée en une matière à mémoire de forme, de sorte que l'on peut lui donner une forme bombée adaptée au cas par cas pour épouser au mieux la forme occipitale de la personne à laquelle est destiné chaque dispositif appui-tête particulier.

Le repose-tête 1 est mobile en basculement d'avant en arrière par rotation au niveau de sa plaque interne 10 autour d'un axe d'articulation transversal matérialisé par une tige 12 dont les extrémités sont retenues à travers des oreillettes en saillie de la pièce 10 qui se situent fixes en proéminence sur l'arrière du repose-tête en partie basse de celui-ci. Cette tige fait axe de charnière sur un embout 14 à pan coupé qui termine le coulisseau 3, comme le montrent les figures 1 et 2.

Les mouvements du repose-tête 1 en rotation autour de l'axe d'articulation, ou axe charnière, s'exercent à l'encontre d'un ressort de torsion 13 enroulé autour de la tige 12 qui prend appui par l'une de ses pattes d'extrémité sur l'arrière de la plaque 10 interne au repose-tête et dont l'autre patte d'extrémité se situe en appui sur l'embout 14. Le sens d'enroulement des spires du ressort sur l'axe tige 12 est défini de sorte à obtenir la torsion du ressort quand le repose-tête s'incline vers l'arrière, lequel est ainsi sollicité à revenir vers l'avant dans sa position nominale initiale.

Une vis de réglage 11 permet de fixer l'inclinaison initiale du repose-tête. Cette vis traverse le coulisseau 3 en sa partie centrale jusqu'à une butée appropriée formée fixe sur l'arrière du repose-tête, et plus précisément ici jusqu'à buter sur la plaque interne 10, sur sa face arrière. Elle est à tête moletée, ce qui permet à l'utilisateur portant le dispositif déjà sur lui de la manoeuvrer pour régler la force du ressort en fonctionnement.

Le support dorsal 2 forme un corps principal 5, se plaçant allongé verticalement sur la colonne vertébrale du porteur en fonctionnement, et deux ailes symétriques terminée par des fentes 21 et 22 servant à accrocher les bretelles du harnais. Le corps 5 est creux en sa partie supérieure, pour recevoir à coulissement le coulisseau 3. Il contient un ressort de compression dont on voit apparaître les spires en 4 sur la figure 1.

Ce ressort fait partie comme le ressort de torsion 13 des moyens de rappel élastique qui rappellent le repose-tête vers sa position nominale à l'encontre des efforts exercés dessus par le porteur du dispositif lors de ses mouvements de tête en extension. Les deux ressorts agissent simultanément l'un sur l'élongation du support dorsal qui fixe la position en hauteur de cou de la liaison articulée du repose-tête sur le support dorsal, l'autre sur son inclinaison autour de l'axe d'articulation.

Plus précisément, le ressort de compression 4 est contenu guidé entre des portées opposées prévues l'une sur le coulisseau 3, l'autre dans le support dorsal 2 en sa partie basse, pour former des butées entre lesquelles le ressort est plus ou moins comprimé quand la distance entre les deux butées varie. Bien que cela ne soit pas représenté en détails sur la figure, on peut comprendre, à titre d'exemple, que le coulisseau 3 présente un alésage longitudinal dans lequel le ressort pénètre vers le haut jusqu'à un fond sur lequel il vient en butée en son extrémité supérieure et que les déplacements du coulisseau 3 dans le support dorsal 2 peuvent avantageusement être guidés par glissement d'ergots dans des lumières longitudinales coopérantes.

La position initiale du coulisseau 3 dans le support dorsal 2 est réglable au moyen d'une vis, vissée dans la matière du coulisseau, qui sert à l'enfoncer plus ou moins, en modifiant ainsi la position de la butée haute du ressort 4. Cette vis est manoeuvrable par l'utilisateur, au moyen d'une tête moletée 15 qui lui est accessible à travers une fenêtre ménagée dans le coulisseau (fig. 1). C'est ainsi que l'on peut régler la position que prend la charnière d'articulation du repose-tête dans sa position dite nominale. Une position initiale peut être réglée pour adapter le dispositif appui-tête à la longueur de cou de son utilisateur habituel et des réglages plus fins peuvent être commandés par l'utilisateur pendant qu'il porte le dispositif.

Les effets du ressort de compression sont eux-mêmes réglables par ailleurs. A cet effet, le dispositif décrit ici en exemple comporte des moyens de déplacement de la butée sur laquelle le ressort prend appui en son extrémité basse, à l'opposé de son extrémité en butée sur le coulisseau. C'est ainsi que l'on voit sur la figure 1 la tête moletée 40 d'une vis sans fin interne au corps 5 du support dorsal qui engrène avec un écrou intégré avec la portée faisant butée basse pour le ressort de compression. Cet écrou (ou un trou taraudé équivalent) est maintenu immobilisé en rotation. Il présente en effet un ergot 41 qui reste guidé dans une fente verticale 20 du corps du support dorsal. En agissant sur la tête de vis 40, accessible à tout moment en bas de l'appui-tête, on règle le taux de compression initial du ressort. L'ergot 41 étant visible dans la fente 20, il fournit une information visuelle concernant la raideur du ressort de compression 4.

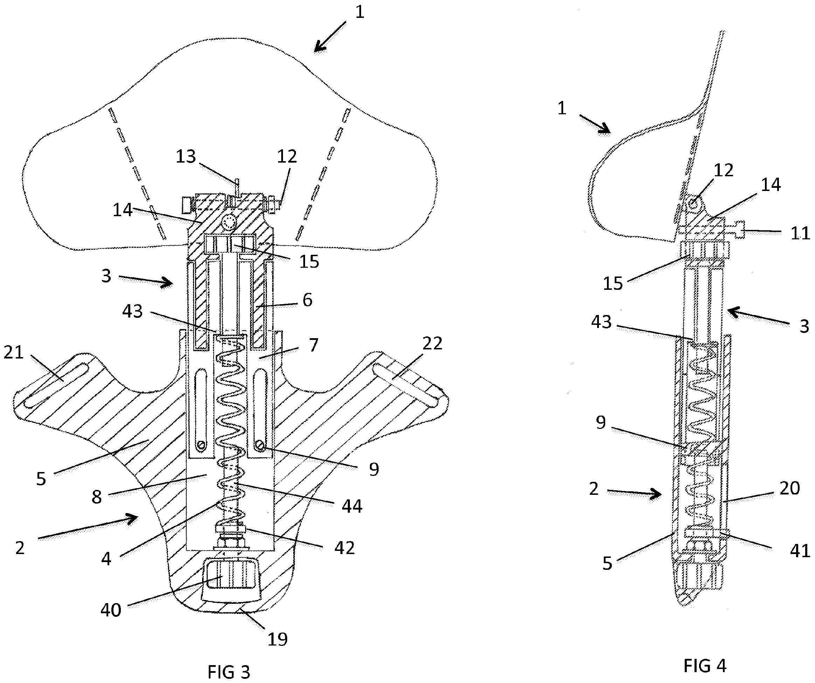

En faisant maintenant référence aux figures 3 et 4, on décrira plus en détail une variante de réalisation du dispositif de l'invention dans laquelle le coulisseau 3 est réalisé télescopique en combinaison avec des moyens de réglage de la raideur du ressort de compression sollicitant le repose-tête en déplacement le long du support dorsal dans une déformation d'élongation élastique du support dorsal.

Dans ce mode de réalisation on retrouve les mêmes éléments essentiels que dans la réalisation précédente, dont le repose-tête 1, le support dorsal 2 et le coulisseau 3. La réalisation de ce dernier respecte que le repose-tête est monté élastiquement basculant en rotation autour d'un axe de pivotement 12 transversal à la direction longitudinale du support dorsal 2 grâce au ressort de torsion 13. On notera ici qu'en variante, un montage à basculement élastique du repose-tête similaire pourrait être concrétisé sous forme d'une plaque flexible comme il a été décrit dans le brevet antérieur précédemment cité tout en restant dans le cadre de l'invention. On préfère toutefois le montage à basculement autour d'une tige charnière comme décrit en référence aux figures pour la commodité de réglage qu'il offre.

Le coulisseau 3 se décompose en un élément supérieur 6, terminé par l'embout 14 sur lequel prend appui le ressort 13, et un élément inférieur 7, qui coulisse à l'intérieur du corps 5 du support dorsal. L'élément supérieur est à section annulaire de manière à coulisser longitudinalement dans une cavité réceptrice ménagée dans la matière de l'élément inférieur 7. Plus exactement, la section est semi-annulaire car la face du support dorsal qui se place contre le dos de l'utilisateur est plane.

L'inclinaison initiale du repose-tête est comme précédemment réglable au moyen de la vis 11 à tête moletée dont la pointe appuie sur une partie médiane rigide du repose-tête et qui se visse dans la matière de l'embout 14 terminant l'élément supérieur 6 du coulisseau 3.

A l'intérieur du corps 5 du support dorsal on retrouve aussi un ressort de compression 4 dont l'action s'exerce entre deux butées opposées à l'intérieur d'un alésage 8 ménagé pour le recevoir dans corps 5 du support dorsal et dans le coulisseau 3, plus exactement ici dans l'élément inférieur 6 du coulisseau. La portée interne au coulisseau 3 (considéré dans l'ensemble des deux éléments qui le constitue) qui formée butée d'appui du ressort de compression 4 à son extrémité supérieure est située en 43 au fond dudit alésage. Cette butée haute du ressort de compression est réglable dans sa position axiale en commandant un déplacement relatif de coulissement entre les deux éléments du coulisseau. Ce réglage de la position longitudinale de la butée haute 43 au sein du coulisseau 3 est assuré, manuellement depuis l'extérieur de l'ensemble, au moyen d'une vis qui se visse dans la matière de l'élément inférieur 7 du coulisseau télescopique et dont la tête moletée 15 se loge dans une cavité ouverte de l'élément supérieur 6 du coulisseau dans laquelle elle est libre en rotation, mais fixe en position axiale.

Dans son coulissement relativement au support dorsal, l'élément inférieur 7 du coulisseau télescopique est guidé au moyen d'ergots 9 qui sont fixes dans le corps 5 du support dorsal et se déplacent dans deux lumières coopérantes ménagées verticalement dans la matière de l'élément 7. Le déplacement relatif s'effectue dans les limites de ces lumières, de sorte que le ressort de compression ne peut jamais expulser le coulisseau hors du corps du support dorsal. Le coulisseau 3 dans son ensemble reste efficacement maintenu dans l'axe longitudinal du support dorsal.

La portée d'appui de l'extrémité inférieure du ressort de compression 4 est représentée en 42. Cette butée basse du ressort engrène comme un écrou avec le filet d'une tige filetée 44, formant vis sans fin, autour de laquelle les spires du ressort de compression 4 s'enroulent librement. La tige filetée 44 est montée libre en rotation mais fixe en position longitudinale à travers le corps 5 du support dorsal, et à l'extérieur de celui-ci, elle présente une tête moletée 40 qui permet de commander sa rotation depuis l'extérieur du support dorsal comme décrit précédemment. De même, la butée 42 ne peut être entraînée en rotation avec la tige filetée 44, car elle est maintenue immobile en rotation par son ergot 41, qui est guidé emprisonné dans une fente 20 (fig. 4) ménagée à travers la paroi du corps 5 du support dorsal. De la sorte, en manoeuvrant la tête moletée 40, on règle la position longitudinale de la butée basse du ressort 4 dans le corps 5 du support dorsal.

On décrira maintenant la partie harnais de l'ensemble suivant l'invention après avoir relevé des figures 3 et 4 que dans son extrémité inférieure le corps 5 du support dorsal forme un oeillet d'attache de sangle 19 en plus des deux oeillets latéraux formés par les mêmes fentes 21 et 22 que dans le mode de réalisation de la figure 1.

Les figures 5 et 6 illustrent un mode de réalisation particulièrement avantageux de la partie harnais qui est associée à la partie mécanique dans le dispositif appui-tête selon l'invention. L'ensemble est représenté comme il est porté par son utilisateur, le harnais maintenant alors le support dorsal 2 en haut et au centre du dos de l'utilisateur. Le harnais est attaché de façon symétrique et réciproque, de chaque côté du support dorsal. Il comprend une partie haute à laquelle est attaché le support dorsal 2, complété ici par une partie basse qui participe avec la partie haute pour bien maintenir le support dorsal 2 dans sa position fonctionnelle.

La partie haute du harnais comprend deux bretelles 50 et 51 qui s'attachent chacune aux extrémités respectives d'un coussin lombaire transversal 52. Ces bretelles passent dans les lumières ou fentes 21, 22 des ailes du support dorsal et autour des épaules de l'utilisateur. Elles se serrent à ce niveau au moyen de boucles de serrage 65, en stabilisant ainsi la longueur efficace des bretelles, laquelle est réglable par l'utilisateur en tirant devant lui sur les brins libres des sangles. Dans le dos de l'utilisateur le coussin lombaire 52 est lié directement au support dorsal 2 sur lequel est monté le repose-têtel, par un coussin vertébral 67, tendu par une sangle qui s'attache dans l'œillet inférieur 19 du support dorsale 2.

Le coussin lombaire 52 est relié à la partie basse du harnais, symétriquement par deux sangles latérales (la sangle latérale droite 57 est visible sur la figure 6). La partie basse est constituée de deux boucles 55 et 56 qui viennent se serrer autour des cuisses au niveau du pli de l'aine. A ces deux boucles de tour de cuisse 55 et 56 sont aussi attachées respectivement deux sangles 53 et 54, symétriquement de chaque côté du bassin, qui se croisent sur le devant du porteur (fig. 5) pour venir s'attacher de manière réciproque respectivement sur les bretelles 50 et 51, non pas à la hauteur de la poitrine, mais bien au niveau des épaules, et plus précisément devant les clavicules. Chacune des sangles croisées 53-54 est munie d'un raccord par enclipsage entre brin supérieur et brin inférieur et passe par une boucle de serrage, de sorte que l'utilisateur peut en régler la longueur en tirant sur le brin libre devant lui.

Par le fait notamment qu'elle se croisent sur la poitrine de l'utilisateur, les sangles 53 et 54 ont pour effet de reprendre les efforts s'exerçant dans le dos de l'utilisateur en limitant ainsi la cambrure des lombaires et en évitant donc les traumatismes qui peuvent en résulter. Elles agissent par rappel au niveau des épaules, ce qui a pour effet de renvoyer la force vers l'avant du bassin. Les boucles de tour de cuisse 55, 56 qui passent autour des jambes, permettent d'empêcher le coussin lombaire 52 de remonter. Ceci permet de ne pas avoir à serrer la partie haute du harnais pour que le support dorsal reste dans sa position. La respiration de l'utilisateur est ainsi facilitée.

Une sangle pectorale 59 vient avantageusement compléter la partie haute du harnais. Elle est disposée transversalement sur la poitrine de l'utilisateur et elle s'attache de chaque côté à chacune des deux bretelles 50 et 51 respectivement. Elle est aussi de longueur réglable et faite de deux brins qui se raccordent par enclipsage. Elle sert essentiellement à relier les deux bretelles entre elles en les tirant l'une vers l'autre sur le devant de façon à ce qu'elles ne risquent pas de gêner l'utilisateur par des frottements intempestifs sur les côtes et sous les aisselles comme en entraînent souvent les mouvements des bras.

Quant à la partie basse elle peut comporter des lanières élastiques 69 qui épousent le galbe des fesses en joignant dans le dos de l'utilisateur les sangles de tour de cuisse 55 et 56 respectivement au coussin lombaire 52. Elles évitent que les sangles de tour de cuisse descendent sur les cuisses de l'utilisateur quand il se penche en avant.

La description qui précède explique clairement comment l'invention permet d'atteindre les objectifs qu'elle s'est fixés. Elle fournit un dispositif appui-tête offrant un grand confort pendant une longue durée de port sans induire de fatigue musculo-squelettique, notamment par report des efforts en soulagement des lombaires ainsi que des cervicales et par adaptation fine à la morphologie du porteur (réglages à faire en particulier par une tierce personne sur l'utilisateur) et aux conditions particulières dans chaque situation d'utilisation (réglages accessibles à l'utilisateur, éventuellement par action d'une seule main).

## Revendications

1. Dispositif appui-tête comportant un repose-tête (1) et un support dorsal (2) associé à un harnais de maintien dudit support dorsal sur le dos d'un utilisateur porteur dudit dispositif, dans lequel le repose-tête est monté élastiquement basculant sur le support dorsal en suivi des mouvements en extension de la tête du porteur, **caractérisé en ce qu'**il comporte en outre des moyens (4) d'élongation élastique par lesquels le repose-tête est monté élastiquement coulissant en translation longitudinale le long du support dorsal et est sollicité axialement vers une position nominale de repos à rencontre des mouvements de la tête du porteur tendant à l'enfoncer dans le support dorsal.

2. Dispositif appui-tête suivant la revendication 1 dans lequel le repose-tête est monté mobile en basculement sur le support dorsal par rotation autour d'un axe de rotation (12) formant charnière sur un coulisseau (3) monté coulissant en translation dans un corps longitudinal (5) dudit support dorsal.

3. Dispositif appui-tête suivant la revendication 2 comportant un ressort de torsion (13) disposé autour dudit axe de rotation (12) et prenant appui d'une part sur le repose-tête (1), et d'autre part sur ledit coulisseau (3).

4. Dispositif appui-tête suivant la revendication 2 ou 3 dans lequel lesdits moyens d'élongation élastique du support dorsal comportent un ressort de compression (4) prenant appui sur une butée basse (42) dans le corps (5) du support dorsal et sur une butée haute (43) dans ledit coulisseau (3).

5. Dispositif appui-tête selon la revendication 4, comportant des moyens (15) de réglage d'une position axiale initiale de ladite butée haute (43) dans le coulisseau (3).

6. Dispositif appui-tête selon la revendication 4 ou 5, comportant des moyens (41-42) de réglage d'une position axiale initiale de ladite butée basse (42) dans le corps (5) du support dorsal.

7. Dispositif appui-tête suivant la revendication 1, dans lequel le repose-tête est monté mobile en basculement par rotation autour d'un axe de rotation (12) formant charnière, sur un élément supérieur (6) d'un coulisseau (3) réalisé télescopique, dont un élément inférieur (7), axialement en liaison coulissante avec son élément supérieur, est monté coulissant en translation dans un corps longitudinal (5) dudit support dorsal, et dans lequel des moyens de rappel élastique du repose-tête vers une position nominale sur le support dorsal comportent un premier ressort (13) agissant en antagonisme avec les mouvements de basculement transversal du repose-tête par rapport audit coulisseau, et un second ressort (4) agissant en antagonisme avec les mouvements de translation longitudinale du repose-tête par coulissement dudit coulisseau par rapport audit corps longitudinal du support dorsal.

8. Dispositif appui-tête suivant la revendication 7 dans lequel ledit premier ressort est un ressort de torsion (13) disposé autour dudit axe de rotation (12) et prenant appui d'une part sur le repose-tête (1), et d'autre part sur ledit élément supérieur (6) dudit coulisseau (3) et dans lequel ledit second ressort est un ressort de compression qui est maintenu guidé en butée en son extrémité haute sur une portée ménagée dans l'élément inférieur (7) du coulisseau (3) et en butée en son extrémité basse sur une portée (42) formée dans ledit corps longitudinal (5) du support dorsal.

9. Dispositif appui-tête suivant l'une des revendications 3 et 8, comportant en outre des moyens de réglage de l'inclinaison vers l'arrière du repose-tête par une vis (11) à tête moletée se vissant dans ledit coulisseau jusqu'à une butée formée fixe sur l'arrière du repose-tête.

10. Dispositif appui-tête suivant l'une des revendications 4, 8 et 9, comportant en outre des moyens de réglage d'une dureté initiale du ressort de compression (4) par réglage de la position de la butée ou portée (42) sur laquelle il prend appui en son extrémité basse dans le corps (5) du support dorsal au moyen d'une tige filetée (44) présentant une tête moletée (19) accessible hors le support dorsal qui engrène avec un écrou immobilisé en rotation.

11. Dispositif appui-tête suivant l'une des revendications 5 et 8 à 10, comportant des moyens de réglage d'une position initiale de la butée ou portée (43) sur laquelle ledit ressort de compression (4) prend appui en son extrémité haute dans ledit coulisseau (3) au moyen d'une vis à tête moletée (15) accessible à travers une fenêtre dudit coulisseau.

12. Dispositif appui-tête selon l'une des revendications précédentes, dans lequel le harnais auquel sont associés le repose-tête et le support dorsal comporte d'une part une partie haute comprenant une paire de bretelles (50, 51) s'attachant respectivement des deux côtés du support dorsal qui sont aptes à prendre place sur les épaules de l'utilisateur, d'autre part une partie basse (55, 56) apte à prendre place autour des cuisses de l'utilisateur, la partie haute et la partie basse étant reliées par un coussin lombaire (52) dans le dos d'un utilisateur porteur du dispositif et, sur le devant, par deux sangles ventrales (53, 54) se fixant chacune sur la partie basse du harnais en se croisant avant de s'attacher aux bretelles de ladite partie haute au niveau des épaules.

13. Dispositif appui-tête selon la revendication 12, dans lequel ledit cousin lombaire (52) est lié en partie haute du harnais à un coussin vertébral (67) s'attachant audit support dorsal (2), et en partie basse du harnais, à deux sangles de tour de cuisse (55, 56) symétriquement par deux sangles latérales (57).

14. Dispositif appui-tête selon la revendication 12 ou 13, dans lequel ledit harnais comprend en sa partie haute une sangle pectorale (59) s'attachant des deux côtés respectivement sur chacune des bretelles (50, 51).

## Patentansprüche

1. Kopfstützenvorrichtung, umfassend eine Kopfauflage (1) und eine Rückenstütze (2), die mit einem Gurtwerk zum Halten der Rückenstütze an dem Rücken eines Benutzers, der die Vorrichtung trägt, wobei die Kopfauflage elastisch schwenkbar an der Rückenstütze, Bewegungen folgend, als Erweiterung des Kopfes des Trägers Bewegungen, montiert ist, **dadurch gekennzeichnet, dass** sie weiterhin elastische Verlängerungsmittel (4) umfasst, durch die die Kopfauflage elastisch gleitend, sich längsverschiebend entlang der Rückenstütze montiert ist und axial gegen eine Sollruhestellung gegenüber Bewegungen des Kopfes des Trägers vorgespannt ist, der dazu neigt, sich in die Rückenstütze abzusenken.

2. Kopfstützenvorrichtung nach Anspruch 1, wobei die Kopfauflage beweglich, sich schwenkend auf der Rückenstütze durch Drehung um eine Drehachse (12) montiert ist, wobei ein Gelenk an einem Schieber (3) gebildet wird, der gleitend, translatorisch in einem länglichen Körper (5) der Rückenstütze montiert ist.

3. Kopfstützenvorrichtung nach Anspruch 2, umfassend eine Drehfeder (13), die um die Drehachse (12) angeordnet ist und einerseits auf der Kopfauflage (1) und andererseits auf dem Schieber (3) aufliegt.

4. Kopfstützenvorrichtung nach Anspruch 2 oder 3, wobei die elastischen Verlängerungsmittel der Rückenstütze eine Druckfeder (4) umfasst, die auf einem unteren Anschlag (42) in dem Körper (5) der Rückenstütze und auf einem oberen Anschlag (43) in dem Schieber (3) aufliegt.

5. Kopfstützenvorrichtung nach Anspruch 4, umfassend Mittel (15) zur Einstellung einer axialen Ausgangsstellung des oberen Anschlags (43) in dem Schieber (3).

6. Kopfstützenvorrichtung nach Anspruch 4 oder 5, umfassend Mittel (41-42) zur Einstellung einer axialen Anfangsstellung des unteren Anschlags (42) in dem Körper (5) der Rückenstütze.

7. Kopfstützenvorrichtung nach Anspruch 1, wobei die Kopfauflage beweglich, sich schwenkend durch Drehung um eine Drehachse (12) montiert ist, wobei ein Gelenk an einem oberen Element (6) eines teleskopisch umgesetzten Schiebers (3) gebildet wird, von dem ein unteres Element (7) axial in Gleitverbindung mit seinem oberen Element gleitend, translatorisch in einem länglichen Körper (5) der Rückenstütze montiert ist, und wobei Mittel zur elastischen Rückstellung der Kopfauflage in eine Sollstellung auf der Rückenstütze eine erste Feder (13), die antagonistisch zu den Querschwenkbewegungen der Kopfauflage in Bezug auf den Schieber wirken, und eine zweite Feder (4), die antagonistisch zu den Längstranslationsbewegungen der Kopfauflage durch Verschieben des Schiebers in Bezug auf den länglichen Körper der Rückenstütze wirkt, umfassen.

8. Kopfstützenvorrichtung nach Anspruch 7, wobei die erste Feder eine Drehfeder (13) ist, die um die Drehachse (12) angeordnet ist und einerseits auf der Kopfauflage (1) und andererseits auf dem oberen Element (6) des Schiebers (3) aufliegt, und wobei die zweite Feder eine Druckfeder ist, die im Anschlag mit ihrem oberen Ende auf einer Lageraufnahme in dem unteren Element (7) des Schiebers (3) und im Anschlag mit ihrem unteren Ende auf einer Aufnahme (42), die in dem länglichen Körper (5) der Rückenstütze ausgebildet ist, geführt gehalten wird.

9. Kopfstützenvorrichtung nach einem der Ansprüche 3 und 8, weiterhin umfassend Mittel zur Einstellung der Neigung zur Rückseite der Kopfauflage durch eine Rändelkopfschraube (11), die in den Schieber bis zu einem Anschlag, der fest auf der Rückseite der Kopfauflage ausgebildet ist, geschraubt wird.

10. Kopfstützenvorrichtung nach einem der Ansprüche 4, 8 und 9, weiterhin umfassend Mittel zur Einstellung einer Anfangshärte der Druckfeder (4) durch Einstellung der Stellung des Anschlags oder der Aufnahme (42), auf dem bzw. der sie mit ihrem unteren Ende in dem Körper (5) der Rückenstütze aufliegt, mittels einer Gewindestange (44), die einen Rändelkopf (19) aufweist, auf den von außerhalb der Rückenstütze zugegriffen werden kann, die mit einer Mutter in Eingriff kommt, die drehimmobilisiert ist.

11. Kopfstützenvorrichtung nach einem der Ansprüche 5 und 8 bis 10, umfassend Mittel zur Einstellung einer Anfangsstellung des Anschlags oder der Aufnahme (43), auf dem bzw. der die Druckfeder (4) mit ihrem oberen Ende in dem Schieber (3) aufliegt, mittels einer Rändelkopfschraube (15), auf die durch ein Fenster des Schiebers zugegriffen werden kann.

12. Kopfstützenvorrichtung nach einem der vorhergehenden Ansprüche, wobei das Gurtwerk, mit dem die Kopfauflage und die Rückenstütze verbunden sind, einerseits einen oberen Teil, umfassend ein Paar von Schulterriemen (50, 51), die jeweils an den beiden Seiten der Rückenstütze angebracht werden und die dazu geeignet sind, auf den Schultern des Benutzers aufzuliegen, und andererseits einen unteren Teil (55, 56), der dazu geeignet ist, um die Schenkel des Benutzers aufzuliegen, umfasst, wobei der obere Teil und der untere Teil durch ein Lendenpolster (52) im Rücken eines Benutzers, der die Vorrichtung trägt, und an der Vorderseite durch zwei ventrale Gurte (53, 54), die jeweils am unteren Teil des Gurtwerks befestigt werden, indem sie überkreuzt werden, bevor sie an den Schulterriemen des oberen Teils auf Höhe der Schultern angebracht werden, miteinander verbunden sind.

13. Kopfstützenvorrichtung nach Anspruch 12, wobei das Lendenpolster (52) am oberen Teil des Gurtwerks mit einem Wirbelpolster (67), das an der Rückenstütze (2) angebracht wird, und am unteren Teil des Gurtwerks mit zwei Oberschenkelgurten (55, 56) symmetrisch durch zwei laterale Gurte (57) verbunden ist.

14. Kopfstützenvorrichtung nach Anspruch 12 oder 13, wobei das Gurtwerk in seinem oberen Teil einen Brustgurt (59) umfasst, der jeweils an beiden Seiten an jedem der Schulterriemen (50, 51) angebracht wird.

## Claims

1. Headrest device comprising a headrest (1) and a back support (2) associated with a harness for maintaining said back support on the back of a user wearing said device, wherein the said headrest is mounted elastically tilting on the back support following movements in extension of the head of the wearer, **characterised in that** it further comprises means (4) of elastic extension by which the headrest is mounted elastically sliding in longitudinal translation along the back support and is axially driven to a nominal resting position against movements of the head of the wearer tending to sink it into the back support.

2. Headrest device according to claim 1, wherein the headrest is mounted mobile is tilting on the back support by rotation about an axis of rotation (12) forming a hinge on a slider (3) slidably mounted in translation in a longitudinal body (5) of said back support.

3. Headrest device according to claim 2, comprising a torsion spring (13) arranged about said axis of rotation (12) and bearing, on the one hand, on the headrest (1), and on the other hand, on said slider (3).

4. Headrest device according to claim 2 or 3, wherein said means of elastic extension of the back support comprising a compression spring (4) bearing on a bottom abutment (42) in the body (5) of the back support and on a top abutment (43) in said slider (3).

5. Headrest device according to claim 4, comprising means (15) for adjusting an initial axial position of said top abutment (43) in the slider (3).

6. Headrest device according to claim 4 or 5, comprising means (41-42) for adjusting an initial axial position of said bottom abutment (42) in the body (5) of the back support.

7. Headrest device according to claim 1, wherein the headrest is mounted mobile in tipping by rotation about an axis of rotation (12) forming a hinge, on an upper element (6) of a slider (3) carried out telescopically of which a lower element (7), axially in sliding connection with the upper element thereof, is slidably mounted in translation in a longitudinal body (5) of said back support, and wherein elastic return means of the headrest to a nominal position on the back support comprise a first spring (13) acting antagonistically with the transverse tilting movements of the headrest with respect to said slider, and a second spring (4) acting antagonistically with the longitudinal translation movements of the headrest by sliding of said slider with respect to said longitudinal body of the back support.

8. Headrest device according to claim 7, wherein said first spring is a torsion spring (13) arranged about said axis of rotation (12) and bearing, on the one hand, on the headrest (1), and on the other hand, on said upper element (6) of said slider (3) and wherein said second spring is a compression spring which is maintained guided in abutment in the top end thereof on a bearing surface arranged in the lower element (7) of the slider (3) and in abutment on the bottom end thereof on a bearing surface (42) formed in said longitudinal body (5) of the back support.

9. Headrest device according to one of claims 3 and 8, further comprising means for adjusting the tilting towards the rear of the headrest by a knurled head screw (11) being screwed in said slider until an abutment fixedly formed on the rear of the headrest.

10. Headrest device according to one of claims 4, 8 and 9, further comprising means for adjusting an initial hardness of the compression spring (4) by adjusting the position of the abutment or bearing surface (42) whereon it is bearing at the bottom end thereof in the body (5) of the back support by means of a threaded rod (44) having a knurled head (19) accessible outside the back support that meshes with a nut immobilised in rotation.

11. Headrest device according to one of claims 5 and 8 to 10, comprising means for adjusting an initial position of the abutment or bearing surface (43) whereon said compression spring (4) bears at the top end thereof in said slider (3) by means of a knurled head screw (15) accessible through a window of said slider.

12. Headrest device according to one of the preceding claims, wherein the harness to which are associated the headrest and the back support comprises, on the one hand, a top portion that comprises a pair of shoulder straps (50, 51) being attached respectively on the two sides of the back support which are capable of occurring on the shoulders of the user, on the other hand, a low part (55, 56) capable of occurring around the thighs of the user, the top portion and the bottom portion being connected by a lumbar cushion (52) in the back of a user wearing the device and, on the front, by two chest straps (53, 54) each being fixed on the bottom portion of the harness by crossing before being attached to the shoulder straps of said top portion at the shoulders level.

13. Headrest device according to claim 12, wherein said lumbar cushion (52) is connected at the top portion of the harness to a spinal cushion (67) that is attached to said back support (2), and in the bottom portion of the harness, to two thigh straps (55, 56) symmetrically by two lateral straps (57).

14. Headrest device according to claim 12 or 13, wherein said harness comprises in the top portion thereof, a pectoral strap (59) being attached on the two sides respectively on each one of the straps (50, 51).
